Europäisches Patentamt

European Patent Office

Office européen des brevets

(11) Publication number: **0 329 488**
**A2**

# EUROPEAN PATENT APPLICATION

(21) Application number: **89301622.0**

(51) Int. Cl.4: **G 01 N 21/00**

(22) Date of filing: **20.02.89**

(30) Priority: **19.02.88 GB 8803929**

(43) Date of publication of application:
**23.08.89 Bulletin 89/34**

(84) Designated Contracting States:
**AT BE CH DE ES FR GB GR IT LI LU NL SE**

(71) Applicant: **BRITISH LEATHER CONFEDERATION**
**Leather Trade House Kings Park Road**
**Moulton Park Northampton NN3 1JD (GB)**

(72) Inventor: **Webster, Roderick McLeod**
**34 The Knoll Ashway**
**Brixworth Northants NN6 9HY (GB)**

(74) Representative: **Blake, John Henry Francis et al**
**BROOKES AND MARTIN High Holborn House 52/54 High**
**Holborn**
**London WC1V 6SE (GB)**

(54) Detection of grain damage in skins and leather.

(57) The method comprises contacting a skin during leather manufacture with a pigment that is decomposable or decolourizable without damage to the skin, inspecting the skin to locate grain damage revealed by the pigment, and treating the skin to decompose or decolourize the pigment. Typically the pigment is one which is decomposed by pickle acids used in leather processing.

EP 0 329 488 A2

**Description**

## DETECTION OF GRAIN DAMAGE IN SKINS AND LEATHER

This invention relates to a method of detecting grain damage in skins and leather and compositions for use in the method.

Grain quality and character are the most important determinants of the value and end use of a piece of leather. Damage to the grain may occur during the life of the animal, during removal of the skin or in subsequent processing. Almost all types of grain damage are difficult or impossible to detect and assess accurately in raw and part processed skin, only becoming fully apparent when the skin is dyed and dried almost the last steps in making leather. The tanner is, therefore, frequently forced to redirect and reprocess some skins for lower quality productions, incurring considerable financial loss. A method of visualising grain damage in part processed skins, allowing sorting and assignment to appropriate product lines would, therefore, be a great benefit.

In the past when grain damage was suspected, it has been the practice to apply pigments such as iron oxide or carbon black to the skins; when rubbed in, such pigments lodge in damaged areas rendering them visible. However, these pigments persist in the leather and commonly affect the shade produced on dyeing. Similar attempts have been made with dyes and also with optical brighteners, although the latter requires scanning with ultra-violet light, as disclosed in US Patent No.4548609. Although optical brighteners are invisible to the naked eye, their presence on the leather is undesirable since they too may interfere with subsequent dyeing, either physically or by occupying binding sites required by the dye itself.

The present invention overcomes the disadvantages of the prior art by employing pigments which are removable during subsequent processing so that grain quality can be assessed at the earliest stages of production in the factory, without any consequent adverse effect on final leather quality.

According to one aspect of the present invention there is provided a method of detecting grain damage in skins and leather which comprises contacting a skin with a pigment that is decomposable or decolourizable without damage to the skins, inspecting the skin to locate grain damage revealed by the pigment, and treating the skin, preferably by chemical treatment, to decompose or decolourize the pigment.

Typically, suitable pigments may be decomposed or decolourized by chemical treatment as with acid or alkali. In a preferred embodiment, acid-sensitive pigments are used, preferably pigments that are sensitive to acids commonly used in leather processing, such as pickle acids. Suitable acid sensitive pigments are the pigments known generally as ultramarines.

According to a further aspect the invention comprises contacting skins still containing pickle acid with a pigment that is decomposable or decolourizable by an acid, inspecting the skin to locate grain damage revealed by the pigment, and allowing the pickle acid to decompose or decolourize the pigment.

The ultramarines constitute a range of natural or synthetic pigments available in pink (CI Pigment Red 259), violet (CI Pigment Violet 15), green (CI Pigment Green 24) and yellow (CI Pigment Yellow 31) as well as blue (CI Pigment Blue 29), all colours being unstable to acid, The pigment may be diluted with a carrier material, preferably with an acid labile insoluble or sparingly soluble diluent such as calcium or magnesium carbonate. The dilution level is not critical and effective formulations have been found across a wide range of from 5 parts pigment : 95 parts diluent to 3 parts pigment : 2 parts diluent.

The particle size distribution of the pigment composition is important in relation to the highlighting of damage, since large particles cannot penetrate small lesions. The particle sizes of components of the formulation will usually lie in the range of 0.3 to 600 microns. Simple trial experiments will reveal the appropriate size for particular applications.

The pigment formulation may be applied as a dispersion in saline solution or in water, by dipping the skin into the dispersion or by applying it to the skin by hand or mechanical means such as a conveyor with overhead spray or in a tannery drum or similar rotating or tumbling process vessel. Application may be made to any wet leather or part processed goods of suitable pH value, with damage assessment carried out by eye or by a suitable automatic device such as an image analyser. After assessment has been made, treating the goods with pickling acids known in the act but preferably hydrochloric or formic acid will completely remove the pigment and diluent by decomposition and solubilisation. The substrate may then be processed conventionally, being dyed and finished with no interference from solid residues. Hydrogen sulphide produced by acid decomposition of the pigment may be destroyed with the aid of a suitable additive before discharge of the wash liquors, or by incorporation of such an additive in the liquor in which pigment destruction is carried out. Suitable additives include oxidising agents such as sodium hypochlorite or hydrogen peroxide.

For hides and skins at certain process stages, some adjustment of pH may be required to prevent pigment descolourization by acid leaching from the interior of the substrate. Such adjustment may be carried out prior to or concurrently with pigment application using agents such as magnesium oxide, aqueous ammonia, sodium carbonate, ammonium hydrogen carbonate, or suitable proprietary compositions, such as Neosyn EE (Hodgsons Chemicals Ltd).

Another aspect of the invention is the use of a pigment that is decomposable or decolourizable, preferably by an acid, in the manufacture of a composition for contacting with skins or leather to detect grain damage

In a further aspect of the invention, there is provided a defect-revealing composition comprising a pigment that is decomposable or decolourizable, preferably by an acid, optionally dispersed in an acid-labile solid diluent or an inert liquid diluent, typically water or brine.

The composition is preferably in the form of a concentrate for dilution before use, and may contain conventional additives such as thickeners, wetting agents and preservatives.

The invention is illustrated by the following examples:-

## Example 1

### Sorting in Lime

Raw hides cured by various methods (dried, dry salted, wet salted, brined) were soaked back using appropriate conventional systems.

They were then limed in 100% w/w liquor containing 3% hydrated lime, 2.5% sodium sulphide flake, 1% sodium hydrosulphide flake (all percentages on soaked hide weight) and 0.5% w/v of a pigment formulation containing 3 parts magnesium carbonate : 2 parts Ultramarine Blue (Cl Pigment Blue 29) in 16 parts of an aqueous dispersion containing suitable thickener, wetting agent and preservative, (Trade Name Sortassist, commercially available from Hodgson Chemicals Ltd, P O Box 7, Chantry Lane, Beverley, North Humberside, HU17 ONN, England).

On completion of liming/unhairing the stock was unloaded and the grain inspected for damage highlighted by the pigment. On returning the goods to normal processing, the pigment was destroyed in the pickle.

## Example 2

### Sorting of Limed Goods

Bovine hides were limed in the conventional manner with calcium hydroxide, sodium sulphide and sodium hydrosulphide. The lime liquor was drained and the hides refloated with 120% w/w fresh water. Sufficient pigment formulation (as in example 1 above) to give an 0.5% w/v suspension was added and the hides drummed for 15 min and drained.

The hides were unloaded and the grains inspected for damage highlighted by the pigment. After sorting the hides were returned to normal processing, during which the pigment was destroyed in the pickle.

## Example 3

### Sorting of Limed Stock with Surface Deliming

3(a) Limed bovine hides were surface delimed by drumming for 20 mins in 100 w/w liquor containing 0.088% w/v solution of ammonium sulphate. The hides were drained, drummed with 100% w/w liquor containing an 0.5% w/v suspension of the pigment formulation (as in example 1 above) for 15 mins and unloaded. They were then inspected for grain damage highlighted by the pigment, and returned to normal processing. The pigment was destroyed during pickling.

3(b) Limed bovine hides were surface delimed by drumming for 20 min in 100 % w/w liquor containing 0.088% w/v solution of ammonium sulphate. Pigment formulation (as in example 1 above) was added to give an 0.5% w/v suspension in the liquor and the hides drummed for 15 mins. They were then unloaded and inspected for grain damage highlighted by the pigment before being returned to normal processing during which the pigment was destroyed in pickling.

## Example 4

### Sorting of Delimed Goods (Bovine and Ovine)

Bovine and Ovine raw stock were processed appropriately to the delimed state by conventional methods using calcium hydroxide, sodium sulphide and sodium hydrosulphide for unhairing and liming and ammonium sulphate (bovine) or ammonium chloride (ovine) for deliming. The delimed goods were drummed in 100% (on limed wt) liquor containing an 0.5% w/v suspension of pigment formulation (as in example 1 above) for 20 mins at pH 8.5, 30°C and unloaded. The goods were inspected for grain damage highlighted by the pigment, after which they were returned to normal processing and the pigment destroyed in pickling.

## Example 5

### Sorting of Bated Goods (Bovine and Ovine)

Bovine and ovine skins were unhaired, limed and delimed in the appropriate way (see Example 4 above). The skins were then bated in 100% w/w (nn limed wt) liquor for 90 mins at pH 8.5, 35°C with the appropriate offer of a pancreatic bating enzyme, and washed for 15 mins in cold water to inhibit further enzyme action.

The bated hides or skins were then drummed for 15 min in 100% w/w (on limed wt) liquor containing an 0.5% w/v suspension of pigment formulation (as in example 1 above), unloaded, and inspected for grain damage highlighted by the pigment. On returning the hides or skins to normal processing, the pigment was destroyed during pickling.

## Example 6

### Sorting of Pickled Pelts after Pigment Application by Hand

Four dozen pickled sheepskins (2 doz imported storage pickled skins and 2 doz skins freshly pickled after fellmongering) were rinsed in 10% brine for 20 min, finishing at pH 2.0. Each skin in turn was then treated with 30-50 ml of an ultramarine blue (Cl Pigment Blue 29) calcium carbonate pigment preparation(5:95 by weight) diluted 1:10 (by weight) in 10% brine. The applied pigment was rubbed manually into the skin for up to one minute, the skin rinsed in 10% brine and again rubbed for up to one minute. The skin was then examined visually and a record made of the type and distribution of defects shown by the pigment. The skins were then washed for 30 min in 10% brine containing 0.25% v/v hydrochloric acid. Further processing to the dyed leather was carried out as normal and the skins re-assessed; comparison of the two assessments showed that 96% of the grain damage apparent in the dyed skins had been detected in the pickle by application of the pigment formulation No residue from the pigment preparation could be detected in the dyed skins.

Example 7

Sorting of Pickled Pelt after Pigment Application by Spray

Three dozen pickled sheepskins, a mixed pack as in Example 6, were rinsed in 10% brine for 20 min and then passed individually on a conveyor under a spray of the ultramarine blue/calcium carbonate preparation used in Example 6 diluted with 10% brine (3 parts by weight pigment preparation to 97 parts by weight brine). The applied pigment was rubbed into the skins for up to one minute and they were then assessed for type and distribution for damage. After storage for three weeks the skins were treated with 10% brine containing 0.25% v/v hydrochloric acid for 30 min and subsequently processed as normal to dyed leather where they were again examined. Comparison showed that in this case 76% of the grain damage shown by the dyed leather had been visualised using the pigment preparation. No residues from the preparation were visible in the finished leather.

Example 8

Sorting of Depickled Pelts

8(a) Pickled ovine pelts were depickled in 100% w/w (on pickle wt) liquor containing 0.75% w/v sodium hydrogen carbonate and 10% w/v sodium chloride for 40 mins. At this point the liquor pH was 6.0, and the pelts showed a uniform cross-section to bromo-cresol green. The pelts were drained and then drummed with 120% (on pelt wt) of liquor containing an 0.33% w/v suspension of pigment formulation (as in example 1 above) for 15 mins. The pelts were unloaded, and inspected for grain damage highlighted by the pigment. After sorting the pigment was destroyed by repickling the pelts in 100% w/w liquor containing 10% w/v sodium chloride with 2% w/w formic acid (on pelt).

8(b) Pickled ovine pelts were depickled as in example 8a above. Pigment formulation (as in example 1 above) was added to the drum at 0.33% w/v on existing liquor, and the pelts drummed for 15 mins. The pelts were unloaded and inspected for grain damage highlighted by the pigment. After pickling the pigment was destroyed by repickling in 100% w/w liquor containing 10% w/v sodium chloride with 2% w/w formic acid (on pelt).

Example 9

Sorting of Pickled Pelt after Concurrent Depickling/Pigmentation

Storage pickled ovine pelts (pH of expressed liquor 0.8) were drummed in 10% w/v brine (100% w/w on pelt) containing 0.5% w/v sodium hypochlorite solution (14% available chlorine) 0.1% w/v non-ionic detergent, 0.75% w/v Tanbase (Magnesium oxide : trademark of Steetley Quarry Products Ltd and 2% w/v pigment formulation (as in example 1 above) for 30 mins.

The pigmented pelts were unloaded and inspected for grain damage highlighted by the pigment. The pigment remained unbleached after 48 h, after which time the pelts were repickled in 10% w/v brine (100% w/w on pelt) with 2% w/w formic acid.

Example 10

Sorting of Wet Chrome Tanned Leathers after Pigment Application by Hand

Six unsammed bovine sides of wet blue (wet chrome tanned leather) were each treated with 250 ml of the ultramarine blue/calcium carbonate preparation used in Example 6 diluted with 10% brine (one part by weight pigment preparation: four parts by weight brine). The pigment preparation was rubbed into the skins for up to 5 mins and then washed off with a hose. The damage was assessed visually and its type and distribution for each skin recorded, after which the skins were washed with a solution of 0.25% w/v hydrochloric acid. The hides were processed to the dyed leather in a normal manner and the damage visually re-assessed. Comparison of the two assessments showed that 88% of the defects visible in the dyed leather had been detected by pigment application in the wet blue.

Example 11

Sorting of Wet Chrome Tanned Leathers after Pigment Application by Spray

Wet blue leathers were sprayed with a 5% w/v suspension of pigment formulation (as in example 1 above) then slicked out with a metal or rubber slicker to remove surplus colour.

The leathers were then sorted, using the grain damage highlighted by the pigment. Prior to further processing the pigment was bleached by drumming the leathers in 100% w/w water containing 0.5% w/v sodium hypochlorite solution (14% available chlorine) and 2% w/v formic acid for 15 mins.

Example 12

Sorting of Wet Chrome Tanned Leathers after Pigment Application in the Drum

Wet blue ovine leathers were drummed for 20 min in 100% w/w water continuing 2% w/v pigment formulation (as in Example 1 above). They were then unloaded and inspected for grain damage highlighted by the pigment. After sorting the pigment was bleached according to the procedure given in Example 11 above.

## Example 13

### Sorting of Wet Chrome Tanned Leathers after Pigment Application and Concurrent Neutralisation in the drum

Wet blue bovine leather were drummed for 30 min in 100% w/w water containing 0.75% w/w ammonium hydrogen carbonate and 0.8% w/v pigment formulation (as in example 1 above). They were then unloaded and inspected for grain damage highlighted by the pigment. After sorting the pigment was bleached according to the procedure given in Example 11 above.

## Example 14

### Sorting of E.I. Kips after Pigment Application in the Drum

14(a) E.I. kips were washed for 30 mins in water (1000% w/w on dry leather) and drained. They were then drummed for 15 min with 100% w/w of water containing 3% w/v pigment formulation (as in example 1 above), drained and unloaded. The kips were inspected for grain damage highlighted by the pigment.

14(b) E.I. kips were drummed for 30 min in 800% w/w (on dry wt) water containing 0.4% w/v pigment formulation (as in example 1 above). The drum was drained and the goods unloaded. The kips were inspected for grain damage highlighted by the pigment.

## Example 15

### Sorting of E.I. Kips after Pigment Application by Dipping

Dry E.I. kips were dipped in a 1% w/v suspension of pigment formulation (as in example 1 above), and sorted using the grain damage highlighted by the pigment. The dipping solution required periodic agitation (ca. every 60 min) to prevent excessive settling.

## Example 16

### Sorting of Pigmented Depickled Pelts using Image Analysis

Ovine skins were depickled and pigmented as described in Example 8b above.

The pigmented skins were examined using an image analyser; pigmented areas were readily visible on the enhanced and processed image; appropriate analysis allowed the computer to sort skins automatically.

After sorting, the pelts were repickled and the pigment destroyed.

## Example 17

### Sorting of Pigmented Wet Blue Leather using Image Analysis

Ovine wet blue leathers were prepared and drum pigmented as described in Example 12 above.

The pigmented skins were examined using an image analyser; pigmented areas were readily visible on the enhanced and processed image; appropriate analysis allowed the computer to sort skins automatically.

After sorting, the pigment was bleached with formic acid and sodium hypochlorite as described in Example 11 above, and the skins returned to normal processing.

## Claims

1. A method of detecting grain damage in skins and leather which comprises contacting a skin with a pigment that is decomposable or decolourizable without damage to the skin, inspecting the skin to locate grain damage revealed by the pigment, and treating the skin to decompose or decolourize the pigment.

2. A method according to Claim 1 wherein the pigment is decomposed or decolourized by chemical treatment.

3. A method according to Claim 1 or 2 wherein the pigment is dispersed in a solid or liquid diluent.

4. A method according to Claim 1, 2 or 3 wherein the pigment is decomposed or decolourized by treatment with acid or alkali.

5. A method according to Claim 4 wherein the pigment is sensitive to acids commonly used in leather processing.

6. A method of detecting grain damage in skins and leather which comprises contacting skin still containing pickle acid with a pigment that is decomposable or decolourizable by an acid, inspecting the skin to locate grain damage revealed by the pigment, and allowing the pickle acid to decompose or decolourize the pigment.

7. A method according to Claim 4, 5 or 6 wherein the pigment is an ultramarine pigment.

8. A method according to Claim 5, 6 or 7 wherein the pigment is dispersed in an acid labile solid diluent or an inert liquid diluent.

9. A defect-revealing composition comprising a pigment that is decomposable or decolourizable by an acid, dispersed in an acid-labile solid diluent and/or an inert liquid diluent, and optionally containing one or more thickeners, wetting agents and/or preservatives.

10. A composition according to Claim 9 comprising an ultramarine pigment dispersed in a powdered carbonate and/or water or brine.

11. The use of a pigment that is decomposable or decolourizable for the manufacture of a composition for contacting with skins or leather to detect grain damage.

12. The use according to Claim 11 wherein the composition is manufactured from the components defined in Claim 9 or 10.